# EUROPÄISCHE PATENTSCHRIFT

(11) **EP 1 890 750 B1**
(45) Veröffentlichungstag und Bekanntmachung des Hinweises auf die Patenterteilung: **04.02.2009**
(21) Anmeldenummer: 06721920.4
(22) Anmeldetag: 20.04.2006
(51) Int. Cl.: A61M 5/315, A61M 5/24

(54) **LÄNGENAUSGLEICH FÜR EINE EINSTELLVORRICHTUNG EINER INJEKTIONSVORRICHTUNG**
LONGITUDINAL COMPENSATION FOR AN ADJUSTING DEVICE OF AN INJECTION DEVICE
COMPENSATION DE LONGUEUR POUR UN DISPOSITIF DE REGLAGE D'UN DISPOSITIF D'INJECTION

(30) Priorität: 25.04.2005 DE 102005019428
(43) Veröffentlichungstag der Anmeldung: 27.02.2008
(73) Patentinhaber: TecPharma Licensing AG, 3401 Burgdorf (CH)
(72) Erfinder: BURREN, Stefan, CH-3047 Bremgarten (CH); MOSER, Ulrich, CH-3412 Heimiswil (CH); SCHRUL, Christian, CH-3400 Burgdorf (CH)
(86) Internationale Anmeldenummer: PCT/CH2006/000218
(87) Internationale Veröffentlichungsnummer: WO 2006/114010

(56) Entgegenhaltungen:
- WO-A-03/086512
- WO-A-20/05018721
- US-A1- 2004 236 285

## Beschreibung

Die vorliegende Erfindung bezieht sich auf eine Einstellvorrichtung, insbesondere eine Dosiervorrichtung zur Einstellung einer aus einer Injektionsvorrichtung oder einem Pen abzugebenden Dosis, insbesondere auf eine Dosiervorrichtung zur Einstellung einer oder mehrerer fest vorgegebener Dosiseinheiten oder zur Vorbereitung einer Injektionsvorrichtung zur Abgabe einer oder mehrerer fest voreingestellter Dosismengen zum Beispiel aus einer in die Injektionsvorrichtung eingelegten Ampulle, sowie auf ein Verfahren zu deren Herstellung.

Eine Dosiervorrichtung für eine Injektionsvorrichtung ist in der deutschen Patentanmeldung 10 2005 001 159.4 der Anmelderin beschrieben.

Eine Vorrichtung, wie die Erfindung sie betrifft, ist beispielsweise auch aus der WO 97/36626 bekannt. Die Vorrichtung weist ein Gehäuse mit einem Reservoir für das Produkt auf. In dem Reservoir ist ein Kolben aufgenommen, der bei einer Verschiebung in eine Vorschubrichtung das Produkt aus dem Reservoir durch einen Auslass des Reservoirs verdrängt. Eine Zahnstange wirkt als Kolbenstange und schiebt den Kolben in Vorschubrichtung. Im Gehäuse ist ferner ein Antriebsglied relativ zum Gehäuse in und gegen die Vorschubrichtung verschiebbar aufgenommen, das bei einer Verschiebung in Vorschubrichtung die Zahnstange mitnimmt. Hierfür greift das Antriebsglied mit Mitnehmern in Zahnreihen der Zahnstange ein. Zum Einstellen derjenigen Produktmenge, die mit einem Hub verabreicht wird, d.h. durch die Betätigung einer Dosiereinrichtung, wird das Antriebsglied in einer vorderen Stellung um eine eingestellte Dosisweglänge manuell gegen die Vorschubrichtung zurückgezogen. Dabei gleiten die Mitnehmer des Antriebsgliedes über die Zähne der Zahnreihen der Zahnstange und geben dabei elastisch nach. Ein Zurückverschieben der Zahnstange wird durch relativ zum Gehäuse verschiebegesichert aufgenommene Sperrmittel verhindert. Die Sperrmittel wirken mit einer der Zahnreihen der Zahnstange derart zusammen, dass die Sperrmittel eine Verschiebung der Zahnstange gegen die Vorschubrichtung verhindern. Durch elastisches Nachgeben erlauben sie eine Verschiebung der Zahnstange in Vorschubrichtung. Durch die Betätigung des Antriebsknopfes wird mittels des Antriebsglieds die eingestellte Dosisweglänge von der Zahnstange, bzw. dem Kolben, zurückgelegt, sodass die eingestellte Dosis durch den Auslass des Reservoirs ausgeschüttet wird.

Insbesondere wenn aus einer Injektionsvorrichtung kleine genau einzustellende Mengen abgegeben werden sollen, dürfen nur kleine Fertigungstoleranzen auftreten.

Es ist eine Aufgabe der vorliegenden Erfindung eine Einstell- oder Dosiereinrichtung für eine Injektionsvorrichtung vorzuschlagen, welche kostengünstig hergestellt werden kann.

Diese Aufgabe wird durch die Einstell- oder Dosiervorrichtung gemäß den unabhängigen Ansprüchen gelöst. Vorteilhafte Ausfiihrungsformen ergeben sich aus den abhängigen Ansprüchen.

Erfindungsgemäß wird mindestens ein Bauteil einer Einstell- oder Dosiervorrichtung oder einer Injektionsvorrichtung mit mindestens zwei und zum Beispiel auch drei bis zwölf oder mehr als zwölf Eingriffselementen oder Führungen bzw. Gewinden, wie zum Beispiel Nuten, versehen, so dass entsprechende Gegenelemente oder Gewinde eines anderen Bauteils, welche in dieses Bauteil der Injektionsvorrichtung oder Dosiervorrichtung zum Beispiel mittels einer oder mehrerer Nuten eingreifen, in verschiedenen und zum Beispiel radial und/oder axial versetzten Positionen eingesetzt werden können. Da mindestens ein Bauteil der Dosier- oder Injektionsvorrichtung erfindungsgemäß so ausgestaltet ist, dass ein anderes Bauteil in mindestens zwei unterschiedlichen zum Beispiel radial und/oder axial versetzten Einbaulagen eingesetzt werden kann, ist es erfindungsgemäß möglich, bei Einzelteilen, welche zum Beispiel auf Grund von Fertigungstoleranzen zum Beispiel verschieden lang sind, eine Längenkorrektur bei der Montage der Dosiervorrichtung oder der Injektionsvorrichtung durch das Einsetzen eines Bauteils in einer von mehreren möglichen Einsetzpositionen vorzunehmen.

Somit müssen die Einzelteile der Dosier- oder Injektionsvorrichtung nicht mehr mit kleinen Toleranzfenstern hergestellt werden, da die Einzelteile in Abhängigkeit von der jeweiligen tatsächlichen Ausformung und Erfüllung oder Abweichung von einer vorgegebenen Norm erfindungsgemäß in einer Mehrzahl von verschiedenen Orientierungen oder Relativpositionen zusammengefügt werden können, wodurch Fertigungstoleranzen ausgeglichen werden können. Insbesondere bei einer Dosiervorrichtung oder Injektionsvorrichtung, welche zur Einstellung oder Abgabe kleiner Dosisgrößen verwendet werden soll, können Einzelteile mit größeren Toleranzen hergestellt werden, was eine kostengünstigere Fertigung ermöglicht, wobei aufgrund der anschließenden und die jeweils aktuell vorliegende Fertigungstoleranz des Einzelteiles berücksichtigenden Zusammensetzung der Einzelteile die Fertigungstoleranzen ausgeglichen werden können und eine Dosier- oder Injektionsvorrichtung hergestellt werden kann, welche zum Beispiel eine präzise Dosiseinstellung in einem relativ kleinem Bereich ermöglicht, welche bei einem aus dem Stand der Technik bekannten Herstellungsverfahren mit Bauteilen größerer Fertigungstoleranz nicht realisiert werden könnte.

Vorzugsweise wird mindestens ein Bauelement einer Dosiervorrichtung oder einer Injektionsvorrichtung, wie zum Beispiel eine Drehhülse, ein Gehäuse und/oder ein Vorschubglied, mit mindestens zwei und bevorzugt drei bis zwölf oder mehr als zwölf zueinander radial und/oder axial versetzten Gewindegängen versehen. Die Gewindegänge sind bevorzugt als umlaufende Nuten an der Innenseite und/oder Außenseite des jeweiligen Bauelements vorgesehen und können zum Beispiel genau oder mit kleinen Abweichungen um 360°/n zueinander versetzt angeordnet sein, wobei n eine natürliche Zahl größer als 1 ist. Beispielsweise können umlaufende Nuten oder Gewindegänge um 120° oder 60° versetzt zueinander angeordnet sein. Es ist auch möglich die versetzt zueinander angeordneten Gewindegänge nicht gleichmäßig um das Bauelement herum versetzt anzuordnen, das heißt, es kann zum Beispiel ein Gewindegang von einem im Falle der exakten toleranzfreien Fertigung zu verwendenden Norm-Gewindegang um beispielsweise einen ersten Winkel auf eine Seite versetzt zum Norm-Gewindegang angeordnet sein, wobei ein zweiter Gewindegang auf eine entgegengesetzte Seite mit einem von dem ersten Winkel abweichenden zweiten Winkel versetzt angeordnet ist, zum Beispiel um ± 5° oder ± 2°.

Das einem mit mindestens zwei zueinander versetzten Gewindegängen versehene Element zugeordnete und in nicht alle Gewindegänge eingreifende Element der Dosiervorrichtung oder der Injektionsvorrichtung weist mindestens einen, also zum Beispiel auch zwei, drei oder mehr als drei Gewindegänge auf, welche beispielsweise als umlaufende von einem Gewindegrund vorstehende Stege ausgebildet sein können. Erfindungsgemäß sind zwei relativ zueinander verschiebbare und/oder drehbare Elemente so durch ineinander eingreifende Führungen oder Gewinde gekoppelt, dass ein Element in mindestens zwei verschiedenen Relativpositionen oder Führungspositionen in das andere Element eingesetzt werden kann. Beispielsweise kann das Außengewinde einer Drehhülse in einen von mindestens zwei verschiedenen als Nuten versetzt zueinander ausgebildeten Innengewindegänge eines Gehäuses eingesetzt werden, so dass zum Beispiel ein vorderes Ende oder eine Anschlagposition der Drehhülse im vollständig in das Gehäuse eingedrehten Zustand so positioniert ist, dass an einem vorderen Ende des Gehäuses vorgesehene Eingriffselemente oder Schnapper zwischen den Zähnen einer in der Injektionsvorrichtung zum Beispiel durch eine in einer von mehreren möglichen Positionen in die Drehhülse eingesetzte Vorschubhülse geführten Zahnstange zu liegen kommen können, wenn diese beispielsweise von dem in der Drehhülse gelagerten Vorschubglied mittels Schnappern gehalten wird, wodurch vermieden werden kann, dass Schnapper des Gehäuses oder Schnapper der Vorschubhülse in Zwischenpositionen der Zahnstange hängen bleiben oder auf diesen liegen. Liegen jedoch die Schnapper der Vorschubhülse zum Beispiel in einer durch einen Anschlag definierten Position auf den Zähnen der Zahnstange auf, so kann zum Beispiel die Drehhülse und/oder das Vorschubglied in einer anderen möglichen Position eingesetzt werden, um diese Fehlpositionierung zu korrigieren.

Bevorzugt sind die erfindungsgemäß mindestens zwei zueinander versetzten Gewindegänge als Gewindegänge eines Innengewindes oder Außengewindes auf einem ersten Element der Injektionsvorrichtung oder der Dosiervorrichtung vorgesehen, welche mit einem oder einer Mehrzahl von Gewindegängen eines zum Beispiel innerhalb oder außerhalb des erfindungsgemäßen Elements vorgesehenen weiteren Elements zusammenwirken, so dass eine durch das oder die ineinander eingreifenden Gewinde geführte Relativbewegung, wie zum Beispiel eine Drehbewegung, ermöglicht wird.

Allgemein soll unter dem Begriff "Gewinde" im Sinne der Erfindung nicht nur ein vollständig umlaufendes Gewinde beziehungsweise ein Gewindegang verstanden werden, sondern zum Beispiel auch abschnittsweise auf der Innen- oder Außenseite eines Elements vorgesehene Gewindeteilstücke oder Gewindegangsegmente, welche mit einem entsprechenden Gegengewinde durch zum Beispiel das Eingreifen eines Gewindesteges in eine Gewindenut zusammenwirken können.

Gemäß einem weiteren Aspekt bezieht sich die Erfindung auf eine Dosiervorrichtung mit mindestens einem wie oben beschriebenen Element mit mindestens zwei zueinander versetzten Gewindegängen, wobei ein als Nut ausgebildetes umlaufendes oder abschnittförmig vorgesehenes Gewinde eines anderen Elements mit einer der mindestens zwei versetzt zueinander als Nuten vorgesehenen Gewindegänge des Elementes so in Eingriff gebracht werden kann, dass einerseits eine gewünschte Führung eines Gewindes in einem zugeordneten Gewindegang sichergestellt werden kann und andererseits Fertigungstoleranzen beim Zusammenbauen oder Eindrehen eines Bauteils in ein anderes durch die Auswahl einer von mindestens zwei Möglichkeiten zum Eindrehen des Bauteils ausgeglichen werden können, um ein zum Beispiel durch eine Anschlagsposition vorgegebenes Lageverhältnis der Bauteile zueinander zu realisieren.

Vorzugsweise ist das vorgegebene Lageverhältnis bestimmt oder definiert durch einen Abstand, welchen Schnapper oder Eingriffselemente eines Bauelementes von Schnappern oder Eingriffselementen eines anderen mit diesem Bauelement direkt oder indirekt gekoppelten Bauelementes in einer zum Beispiel durch eine vordere Endposition oder einen Anschlag definierten Position haben.

Weiterhin bezieht sich die Erfindung auf ein Verfahren zur Herstellung einer Dosiervorrichtung für eine Injektionsvorrichtung, wobei die Dosiervorrichtung als integraler Bestandteil der Injektionsvorrichtung oder aus separaten Elementen bestehend ausgebildet sein kann, wobei die Dosiervorrichtung mindestens ein zum Beispiel als Innenelement ausgebildetes Einstellelement aufweist, welches in einem zum Beispiel als Außenelement ausgebildeten weiteren Element gelagert und mittels eines Außengewindes zum Beispiel in einem Innengewinde des Außenelements geführt wird, wobei für ein Gewinde des Innen- oder Außenelements, welches zum Beispiel als Steg ausgebildet ist, mindestens zwei zur Führung des Gewindes vorgesehene zum Beispiel als umlaufende Vertiefungen oder Nuten ausgebildete Gewinde in dem entsprechenden Gegenelement vorgesehen sind, so dass das Innenelement auf mindestens zwei verschiedene Arten in das Außenelement eingesetzt, also zum Beispiel eingedreht, werden kann, wodurch ein vorgegebenes Lageverhältnis zwischen dem Innenelement und dem Außenelement hergestellt und zum Beispiel eine Fertigungstoleranz ausgeglichen werden kann.

Das vorgegebene Lageverhältnis kann zum Beispiel durch die Position eines an dem Innenelement oder Außenelement vorgesehenen Schnappers oder Zahneingriffselements zum Beispiel in einer definierten Anschlagsposition festgelegt sein.

Eine Dosiervorrichtung für eine Injektionsvorrichtung kann ein Einstellelement aufweisen, welches zum Beispiel eine zylinderförmige Drehhülse oder ein Knopf sein kann, welcher mit einer zylinderförmigen Drehhülse fest oder drehbar verbunden sein kann. Die Drehhülse weist zum Beispiel mindestens zwei Innengewinde und Außengewinde mit bevorzugt gleichem Drehsinn auf, welche bevorzugt koaxial zueinander angeordnet sind, so dass die Drehhülse in mindestens einem Bereich ein Innengewinde und ein Außengewinde aufweist, welche sich bevorzugt überlappen und zum Beispiel auch etwa über die gesamte Länge der Drehhülse an der Innenseite und der Außenseite des zylinderförmigen Drehhülsenkörpers angeordnet sein können. Die Gewinde sind bevorzugt als Bewegungsgewinde so ausgebildet, dass die Gewinde nicht selbsthemmend sind und können Gewindegänge oder in Gewindegänge eingreifende Elemente oder Nocken oder auch umlaufende Spiralen sein, welche in die Gewinde jeweils gegenüberliegender Gegengewinde eingreifen oder mit diesen zusammenwirken können. Innerhalb und außerhalb der Drehhülse sind Elemente der Injektionsvorrichtung vorgesehen, welche bevorzugt relativ zueinander verdrehgesichert sind oder welche zum Beispiel bezüglich einem Gehäuse der Injektionsvorrichtung verdrehgesichert sind, wobei zum Beispiel ein innerhalb oder außerhalb der Drehhülse an dieser anliegendes Element auch ein Teil des Gehäuses der Injektionsvorrichtung sein kann. Die innerhalb und außerhalb der Drehhülse liegenden Elemente können selbst zum Beispiel zylinderförmig sein oder auch nur entlang eines oder mehrerer Teilbereiche in Umfangsrichtung der Drehhülse ausgebildet sein und weisen Gegengewinde zum Beispiel in Form einzelner Nocken oder umlaufender spiralförmiger Nuten oder Stege auf, welche in die Innen- und Außengewinde der Drehhülse eingreifen oder mit entsprechenden Gewindeelementen der Drehhülse zusammenwirken können. Dabei kann das Innengewinde der Drehhülse die gleiche oder eine andere Steigung als das Außengewinde der Drehhülse aufweisen.

Ist zum Beispiel innerhalb der Drehhülse ein Vorschubglied der Injektionsvorrichtung gelagert, welches sich in einem bestimmten von mehreren möglichen Gewindeeingriffen mit der Drehhülse befindet, so kann eine definierte Bewegung des Vorschubgliedes um eine vorgegebene Distanz in proximale und distale Richtung der Injektionsvorrichtung durch ein Drehen der Drehhülse bewirkt werden, welche in einem bestimmten von mehreren möglichen Gewindeeingriffen mit einem zum Beispiel an der Außenseite der Drehhülse anliegenden Gehäuseteil der Injektionsvorrichtung ist. Hat das Innengewinde der Drehhülse eine kleinere Steigung als das Außengewinde der Drehhülse, so wird, wenn die Drehhülse zum Beispiel um die axiale Länge D aus dem Gehäuse der Injektionsvorrichtung herausgedreht wird, mit welchem die Drehhülse im Gewindeeingriff steht, das Vorschubglied um eine kleinere Distanz d in die gleiche Richtung bewegt, wodurch eine Untersetzung einer Einstellbewegung durch eine kompakte Bauart realisiert werden kann. Somit kann eine Übersetzung eines kleinen funktionalen Weges d in einen größeren Weg D zum einfachen Einstellen zum Beispiel einer Fixdosis realisiert werden.

Ist mit dem Vorschubglied ein Mitnehmer, wie zum Beispiel ein Rastelement verbunden, welches zum Beispiel an einem flexiblen Element oder Arm des Vorschubgliedes vorgesehen ist, so kann ein an dem flexiblen Element vorgesehener zum Beispiel radial nach innen vorstehender Nocken oder Nase über die Zahnung einer als Kolbenstange dienenden Zahnstange oder das Gewinde einer Gewindestange geführt werden, wobei je nach der Größe der axialen Bewegung des Vorschubgliedes relativ zur Zahnstange ein oder mehrere Zähne der Zahnstange durch den elastisch gelagerten Nocken oder Nase überfahren werden, wodurch ein oder mehrere "Klick"-Geräusche erzeugt werden und die aus der Injektionsvorrichtung abzugebende Dosis festgelegt wird. Die so eingestellte Dosis wird durch eine durch ein Zurückschieben oder -drehen der Drehhülse bewirkte Bewegung des Vorschubgliedes oder der Vorschubhülse in distale Richtung, welche durch einen Eingriff der Nocken oder Nasen auf die Zahnstange übertragen wird und an einen unmittelbar oder mittelbar an der Zahnstange anliegenden Stopfen weitergegeben wird, welcher in eine Ampulle zur Verdrängung einer darin enthaltenen Substanz eingeschoben wird, ausgeschüttet.

Bezüglich der prinzipiellen Funktionsweise einer Dosiervorrichtung, welche eine Vorschubhülse oder ein Vorschubglied aufweist, wird auf die deutsche Patentanmeldung mit der Anmeldenummer 10 2004 041 151.4 der Anmelderin verwiesen, deren Offenbarung bezüglich der Ausgestaltung einer Dosiervorrichtung und insbesondere bezüglich des Zusammenwirkens von flexiblen Elementen, welche Nocken oder Nasen tragen und an einer Vorschubhülse und einer Außenhülse oder einem Gehäuse angebracht sind, um mit einer Zahnstange zusammenzuwirken, in diese Anmeldung aufgenommen wird.

Bei den auf der Innen- und Außenseite der Drehhülse vorgesehenen mindestens einen Gewinden mit unterschiedlicher Steigung ist bevorzugt die Steigung desjenigen Gewindes, welches mit dem Vorschubglied im Eingriff steht, kleiner als die Steigung des gegenüberliegenden mindestens einen Gewindes, welches zum Beispiel mit dem Gehäuse oder einem anderen Bauteil der Injektionsvorrichtung im Eingriff steht, bezüglich dessen das Vorschubglied bevorzugt drehsicher gelagert ist, so dass eine bei einem Einstellvorgang durch einen Gewindeeingriff der Drehhülse mit zum Beispiel dem Gehäuse erzwungene Drehung der Drehhülse relativ zu zum Beispiel dem Gehäuse nicht in eine Drehung des ebenfalls im Gewindeeingriff mit der Drehhülse stehenden Vorschubgliedes, sondern in eine Axialbewegung des Vorschubgliedes relativ zum Beispiel zum Gehäuse umgesetzt wird. Dabei kann das Vorschubglied sowohl innerhalb, als auch außerhalb, der Drehhülse mit einem Außen- oder Innengewinde vorgesehen sein. Ebenso ist es möglich, dass die Steigung des Gewindes, mit welchem Drehhülse und Vorschubglied in Eingriff stehen, größer ist als die Steigung des auf der anderen Seite der Drehhülse vorgesehenen Gewindes, wobei in diesem Fall eine kleine Einstellbewegung in eine im Verhältnis dazu größere Axialbewegung des Vorschubgliedes umgesetzt wird.

An der Drehhülse ist vorzugsweise ein Einstellelement, wie zum Beispiel ein Bedienknopf vorgesehen, welcher fest mit der Drehhülse verbunden sein kann, so dass die Drehhülse durch eine Drehung des Bedienknopfes aus der Injektionsvorrichtung herausgedreht werden kann. Ebenso ist es möglich, dass die Drehhülse drehbar mit einem Einstellelement oder Bedienknopf verbunden ist, so dass der Bedienknopf zum Beispiel ohne Drehbewegung aus dem Gehäuse der Injektionsvorrichtung herausgezogen werden kann und die drehbar im Bedienknopf gelagerte Drehhülse aus dem Gehäuse der Injektionsvorrichtung herausgedreht wird.

Vorzugsweise sind die an der Drehhülse vorgesehenen Gewinde nicht selbsthemmend, wobei zum Beispiel der Steigungswinkel des Gewindes so gewählt werden kann, dass der Tangens dieses Steigungswinkels größer als der Reibungskoeffizient der aneinander liegenden und im Gewindeeingriff stehenden Materialien ist. Es können auch Schmiermittel, wie zum Beispiel Teflon, verwendet werden, um nicht selbsthemmende Gewindeeingriffe zu realisieren. Ebenso kann zum Beispiel eine Torsionsfeder vorgesehen sein, welche beim Herausziehen oder- drehen der Drehhülse gespannt oder aufgezogen wird und so zum Beispiel mit der Drehhülse verbunden ist, dass beim Einschieben oder -drehen der Drehhülse die Federkraft in Drehrichtung wirkt, so dass auch selbsthemmende Gewinde verwendet werden können, wobei die Hemmung der Gewinde durch die Feder aufgehoben wird.

Bevorzugt sind die Außen- und Innengewinde der Drehhülse koaxial zueinander angeordnet, d. h. die Gewinde überlappen sich in axialer Richtung, wodurch eine kompakte und die Bauhöhe der Injektionsvorrichtung verringernde Bauweise einer Übersetzung bzw. Untersetzung realisiert werden kann.

Vorzugsweise sind Radial- und/oder Axialanschläge vorgesehen, welche zum Beispiel die Bewegung des Vorschubgliedes und/oder der Drehhülse in radialer und/oder axialer Richtung zum Beispiel relativ zum Gehäuse der Injektionsvorrichtung begrenzen können. Beispielsweise können auf dem Vorschubglied zwei in axialer Richtung voneinander beabstandete Anschläge vorgesehen sein, welche mit einem Anschlagselement des Gehäuses so zusammenwirken, dass das Vorschubglied in axialer Richtung der Injektionsvorrichtung relativ zum Gehäuse nur um eine vorgegebene Distanz d bewegt werden kann, wodurch zum Beispiel eine der Distanz d entsprechende Dosismenge voreingestellt werden kann, die mit der Injektionsvorrichtung abgegeben werden kann. Ebenso können beispielsweise Radialanschläge an der Drehhülse und/oder an den mit der Drehhülse zusammenwirkenden Elementen, also dem Vorschubglied und einem weiteren Element wie zum Beispiel dem Gehäuse vorgesehen sein, welche eine Drehbewegung der Drehhülse begrenzen und zum Beispiel nur eine maximale Drehung von 180° oder beispielsweise zwei volle Umdrehungen ermöglichen. Insbesondere kann mit solchen Anschlägen eine feste Dosis vorgegeben werden, so dass ein Benutzer das Einstellelement zum Beispiel bis zu einem Anschlag eines Elements herauszieht, wobei die voreingestellte Dosis beim Einschieben des Einstellelements abgegeben wird.

Bevorzugt ist an der Drehhülse und/oder an einem mit der Drehhülse verbundenen Einstellelement, wie zum Beispiel einem Bedienknopf, eine Markierung vorgesehen, welche bei einem in die Injektionsvorrichtung eingeschobenen Zustand nicht oder durch zum Beispiel eine Öffnung oder transparentes Material ablesbar ist und welche von einem Benutzer abgelesen oder gesehen werden kann, wenn die Drehhülse und/oder ein mit der Drehhülse verbundenes Bedienelement aus der Injektionsvorrichtung oder dem Gehäuse der Injektionsvorrichtung herausgezogen wurden, so dass es einem Benutzer ermöglicht wird, die aufgrund des Ausschubweges festgelegte eingestellte Dosis abzulesen, die beim Zurückschieben der Drehhülse oder des Bedienknopfes durch die auf die Zahnstange und den Stopfen übertragene Einschubbewegung aus der Injektionsvorrichtung abgegeben wird.

Weiterhin bezieht sich die Erfindung auf eine Injektionsvorrichtung, in welcher eine abzugebende Substanz enthalten ist oder in einer Ampulle eingelegt werden kann und welche eine wie oben beschriebene Dosiervorrichtung aufweist, die mit einem Verdrängungskörper oder Stopfen der Injektionsvorrichtung so gekoppelt werden kann, dass durch die Drehhülse eine abzugebende Dosis eingestellt und ausgeschüttet werden kann.

Die Erfindung wird nachfolgend anhand bevorzugter Ausführungsbeispiele beschrieben. Es zeigen:
- Figuren 1 A bis 1C: eine aus der deutschen Patentanmeldung 10 2005 001 159.4 bekannte Dosiervorrichtung in Grundstellung, beim Einstellen und nach dem Ausschütten einer Dosis;
- Figuren 2A und 2B: die aus der deutschen Patentanmeldung 10 2005 001 159.4 bekannte Dosiervorrichtung im Querschnitt;
- Figur 3: eine Injektionsvorrichtung mit einer aus der deutschen Patentanmeldung 10 2005 001 159.4 bekannten Dosiervor-richtung; und
- Figuren 4A, 4B und 4C: eine erfindungsgemäße Ausführungsform einer Injektionsvorrichtung in Ausgangsstellung, im aufgezogenen Zustand und nach der Ausschüttung; und
- Figur 5: eine Querschnittsansicht entlang der Linie 5-5 in Figur 4.

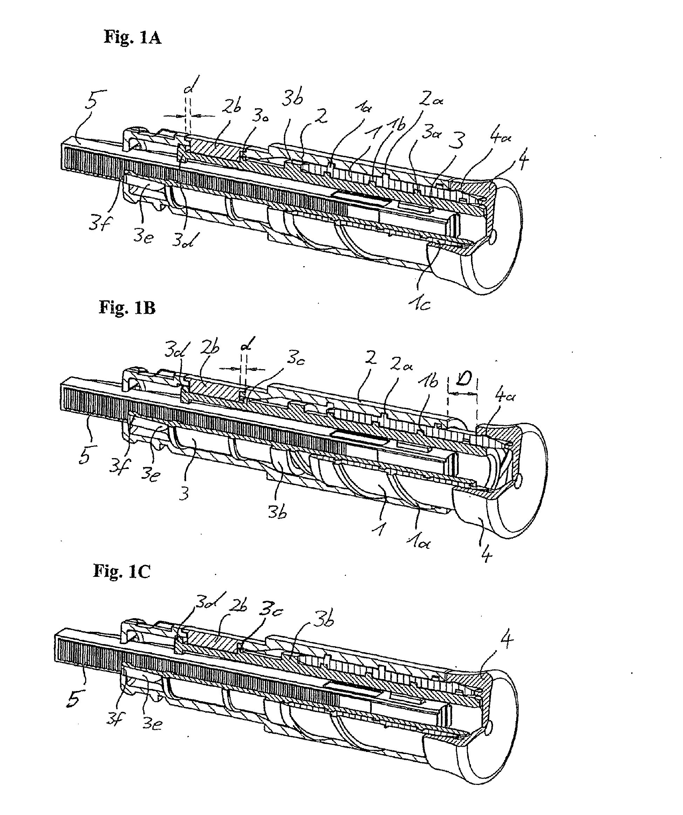

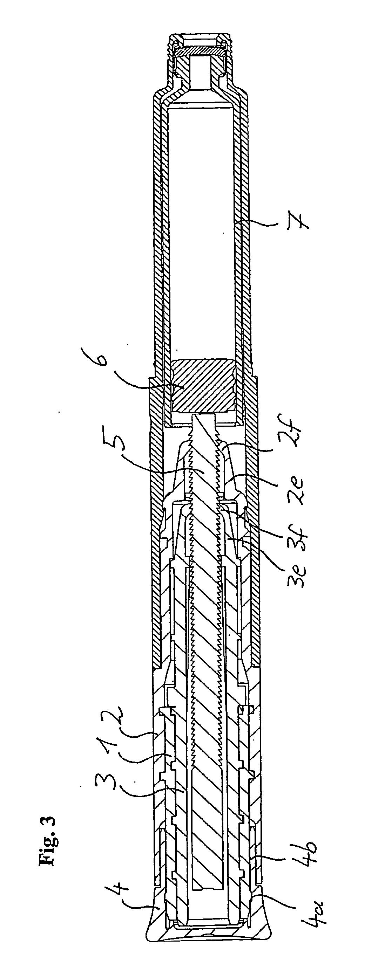

Die Figuren 1A bis 1C zeigen eine aus der deutschen Patentanmeldung 10 2005 001 159.4 bekannte Dosiervorrichtung für eine in Figur 3 beispielhaft gezeigte Injektionsvorrichtung mit einem Gehäuse 2 der Injektionsvorrichtung, welche ein Innengewinde 2a mit einer ersten Steigung von zum Beispiel 49° hat. Koaxial in dem Gehäuse drehbar gelagert ist eine Drehhülse 1 mit einem Außengewinde 1a mit der gleichen Steigung wie das Innengewinde 2a des Gehäuses 2, welches in das Innengewinde 2a eingreift. Die Drehhülse 1 hat ein Innengewinde 1b mit einer Steigung von zum Beispiel 34°, welche kleiner ist als die Steigung des Außengewindes 1a bzw. des Innengewindes 2a des Gehäuses. Der Unterschied der Gewindesteigungen liegt zum Beispiel im Bereich von 10 bis 15 Grad. Innerhalb der Drehhülse 1 ist verdrehgesichert zum Gehäuse 2 ein Vorschubglied 3 gelagert, welches ein Außengewinde 3a hat, das in das Innengewinde 1b der Drehhülse 1 eingreift. An der in Figur 1A links gezeigten distalen oder Vorderseite des Vorschubgliedes 3 sind einander gegenüberliegend zwei an elastischen Armen 3e befestigte Nasen oder Nocken 3f angebracht, welche in die auf der Außenseite einer Zahnstange 5, welche axial verschiebbar innerhalb des Vorschubgliedes 3 angeordnet ist, vorhandene Zahnung eingreifen können. Die Nasen oder Nocken 3f des Vorschubgliedes 3 sind ebenso wie die Nasen oder Nocken 2f, welche mit dem Gehäuse 2 verbunden und in Figur 3 gezeigt sind, in proximaler Richtung relativ zur Zahnstange bewegbar, wobei die Nasen 2f, 3f entgegen die elastische oder Federkraft der elastischen Arme 2e bzw. 3e nach außen gedrückt werden, um über einen oder mehrere Zähne der Zahnstange 5 hinweg bewegt zu werden. Jedoch werden die Nasen 2f und 3f durch die in Richtung auf die Zahnstange 5 wirkende Vorspannung der elastischen Arme 2e und 3e auf die Zahnstange 5 und so in einen Eingriff mit der Zahnung gedrückt, dass eine Bewegung der Nasen 2f und 3f in distale Richtung der Zahnstange 5 nicht möglich ist bzw. durch den Eingriff der Nasen 2f und 3f in die Zahnung der Zahnstange 5 behindert wird.

Am proximalen Ende ist die Drehhülse 1 mit einem Bedienknopf 4 verbunden und drehbar in diesem durch eine auf der Innenseite des Bedienknopfes 4 vorgesehene umlaufende Nut 4a gelagert, in welche ein auf der Außenseite der Drehhülse 1 vorgesehener umlaufender Ring 1c eingreift. Wird der Bedienknopf 4 von einem Benutzer aus dem Gehäuse 2 herausgezogen, so wird von diesem die Drehhülse 1 mitgenommen, welche aufgrund des in das Innengewinde 2a des Gehäuses 2 eingreifenden Außengewindes 1a relativ zum Gehäuse 2 beim Herausziehen gedreht wird. Diese Drehung der Drehhülse 1 wird aufgrund des sich mit dem Innengewinde 1b der Drehhülse 1 in Eingriff befindlichen Außengewindes 3a des relativ zum Gehäuse 2 verdrehsicher gelagerten Vorschubgliedes 3 in eine Axialbewegung des Vorschubgliedes 3 umgesetzt. Aufgrund der größeren Steigung des Außengewindes 1a der Drehhülse im Vergleich zum Innengewinde 1b der Drehhülse wird ein Ausschubweg D, wie in Figur 1B gezeigt, in einen kleineren Ausschubweg d des Vorschubgliedes 3 untersetzt, so dass kleine Dosismengen präzise eingestellt werden können.

Auf der Außenseite des Vorschubgliedes 3 sind zwei radial nach außen vorstehende axial beabstandete Anschläge 3c und 3d vorgesehen, zwischen welchen das Anschlagselement 2b, welches mit dem Gehäuse 2 verbunden ist, eingreift. In der in Figur 1A gezeigten Ausgangsstellung liegt der distale axiale Anschlag 3c des Vorschubgliedes an der distalen Seite des Anschlagselementes 2b an. Der proximale Anschlag 3d des Vorschubgliedes hat einen Abstand d von der distalen Seite des Anschlagselements 2b.

In Figur 1B ist die erfindungsgemäße Dosiervorrichtung nach dem Herausziehen des Bedienknopfes 4 um einen Weg D von zum Beispiel 5 mm gezeigt, was zu der untersetzten Axialbewegung um die Entfernung d von zum Beispiel 0,8119 mm des Vorschubgliedes 3 führt, bis der Anschlag 3d des Vorschubgliedes 3 an der distalen Seite des Anschlagselementes 2b anliegt, wodurch die Ausschubbewegung des Bedienknopfes 4 begrenzt wird. Die Drehhülse 1 wurde dabei zum Beispiel um -90° gedreht.

Beim Herausziehen des Bedienknopfes 4 und der Verschiebung des Vorschubgliedes 3 in proximale Richtung werden die mit dem Vorschubglied 3 verbundenen Nasen oder Nocken 3f in proximale Richtung entlang der durch die mit dem Gehäuse 2 verbundenen Nasen 2f gehaltenen Zahnstange 5 verschoben, so dass die sich einander gegenüberliegenden Nasen 3f zum Beispiel jeweils über einen, zwei oder auch mehr Zähne der Zahnstange 5 nach hinten verschoben werden.

Drückt ein Benutzer auf den Bedienknopf 4 und schiebt diesen wieder zurück in das Gehäuse 2 ein, wie in Figur 1C gezeigt, so wird die Einschubbewegung des Bedienknopfes auf die Drehhülse 1 übertragen, welche sich aufgrund des Gewindeeingriffes mit dem Gehäuse 2 relativ zu diesem um zum Beispiel +90° dreht und durch den Gewindeeingriff mit dem Vorschubglied 3 dieses axial nach vorne schiebt, bis wieder der proximale Anschlag 3c an dem Anschlagselement 2b und die Vorderseite der Drehhülse 1 an einem Anschlag 3b des Vorschubgliedes 3 anliegt. Dabei wird die Zahnstange 5 durch die in die Zahnung eingreifenden Nasen 3f gehalten und zusammen mit dem Vorschubglied 3 in distale Richtung verschoben, wobei die Zahnstange relativ zu dem mit dem Gehäuse 2 verbundenen Nasen 2f um den Weg d verschoben wird, welche nach Beendigung des Vorschubvorganges in Zähne der Zahnstange 5 eingreifen, welche axial in proximale Richtung im Vergleich zur in Figur 1A gezeigten Ausgangsstellung versetzt sind. Diese Vorschubbewegung der Zahnstange 5 wird auf den in Figur 3 gezeigten Kolben 6 übertragen, welcher in die in der Injektionsvorrichtung eingesetzte Ampulle 7 eingeschoben wird und so eine in der Ampulle 7 enthaltene Substanz, wie zum Beispiel Insulin, verdrängt, so dass die der Vorschubbewegung d des Kolbens 6 entsprechende Substanzmenge aus der Ampulle 7 abgegeben wird.

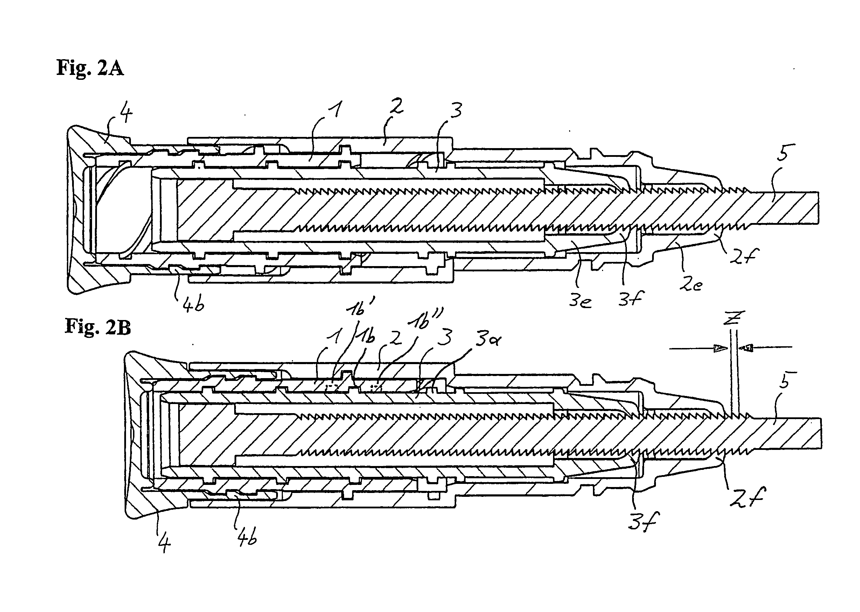

Die Figuren 2A und 2B zeigen im Querschnitt eine weitere Ausführungsform der aus der deutschen Patentanmeldung 10 2005 001 159.4 bekannten Dosiervorrichtung, wobei der Bedienknopf 4 axial in das Gehäuse 2 hineinragende Stege 4b oder auch ein zylinderförmig umlaufendes in eine entsprechende Vertiefung des Gehäuses 2 einschiebbares Element 4b aufweist. Beim Herausziehen des Bedienknopfes 4 ist somit nicht die Außenseite der Drehhülse 1 sichtbar, wie zum Beispiel in Figur 1B gezeigt, sondern die Außenseite des Elementes 4b, wie in Figur 2A gezeigt.

Sowohl auf der Außenseite der Drehhülse 1, als auch auf der Außenseite des Elementes 4b können Markierungen angebracht sein, um einem Benutzer Informationen bezüglich einer aufgrund des Ausschubes aus dem Gehäuse 2 eingestellten Dosis zum Beispiel durch farbige Markierungen oder Ringe anzuzeigen.

Wie zum Beispiel aus den Figuren 2A und 2B gesehen werden kann, kann es bei einem Herstellungsverfahren mit Fertigungstoleranzen im Bereich des Abstandes Z zweier Zähne der Zahnstange 5 zu dem Problem kommen, dass zum Beispiel im in Figur 2B gezeigten eingeschobenen Zustand der Dosiervorrichtung beispielsweise die Zähne der Nasen 3f nicht in die Zwischenräume der Zähne der durch die Nasen 2f gehaltenen Zahnstange 5 eingreifen, sondern zum Beispiel auf den Zähnen der Zahnstange 5 aufliegen. Erfindungsgemäß kann auch ein mit einer größeren Toleranz gefertigtes Vorschubglied 3 dann verwendet werden, wenn zum Beispiel axial versetzt zu dem als umlaufende Nut ausgebildeten Innengewinde 1b der Drehhülse 1 noch ein oder mehrere als umlaufende Nuten 1b' oder 1b" ausgebildete Innengewinde vorgesehen sind, in welche das durch einen umlaufenden Steg gebildete Außengewinde 3a des Vorschubgliedes 3 beim Zusammenbau der Dosiervorrichtung eingeschraubt werden kann, so dass aufgrund des sich ergebenden axialen Versatzes des Vorschubgliedes 3 die Zähne der Nasen 3f wieder in der in Figur 2B gezeigten vorderen Stellung der Dosiervorrichtung in die Zwischenräume zwischen den Zähnen der Zahnstange 5 eingreifen.

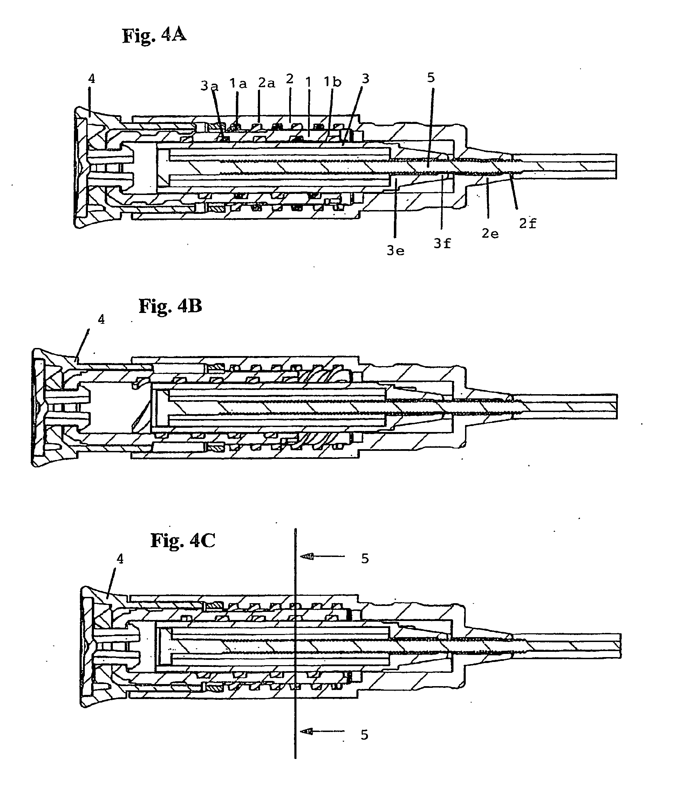

Die Figuren 4A bis 4C zeigen das hintere Ende einer erfindungsgemäßen Injektionsvorrichtung in Grundstellung, in aufgezogenem Zustand und nach der Ausschüttung aus einer in Figur 3 gezeigten Ampulle 7, wobei anhand der flächig schwarz dargestellten Gewindegänge zu erkennen ist, dass die als umlaufende Stege gebildeten Außengewinde 1a und 3a der Drehhülse 1 und des Vorschubgliedes 3 nur in einer aus einer Mehrzahl von vorgesehenen zugeordneten umlaufenden Nuten, welche die Innengewinde 1b und 2a der Drehhülse 1 und des Gehäuses 2 bilden, geführt sind, so dass beim Zusammenbauen der Injektionsvorrichtung Fertigungstoleranzen des Gehäuses 2, der Drehhülse 1 und/oder des Vorschubgliedes 3 so ausgeglichen werden können, dass die Zähne der Schnapper 2f und 3f in gewünschten vorderen und hinteren Positionen der mit dem Knopf 4 gekoppelten Drehhülse 1 immer in Zwischenräume der Zähne der Zahnstange 5 eingreifen.

Wie oben beschrieben, greifen die Schnapper 2f des Gehäuses 2 und die Schnapper 3f der Vorschubhülse 3 in die Verzahnung der Zahnstange 5 und blockieren diese abwechselnd beim Herausziehen beziehungsweise beim Einschieben des Knopfes 4, so dass die Zahnstange 5 nur in distaler Richtung bewegt wird. Dabei koppelt die Drehhülse 1 das Gehäuse 2 und die Vorschubhülse 3 mittels eines ersten Gewindes zwischen dem Gehäuse 2 und der Drehhülse 1 und eines zweiten Gewindes zwischen der Drehhülse 1 und der Vorschubhülse 3 mit bevorzugt unterschiedlicher Steigung.

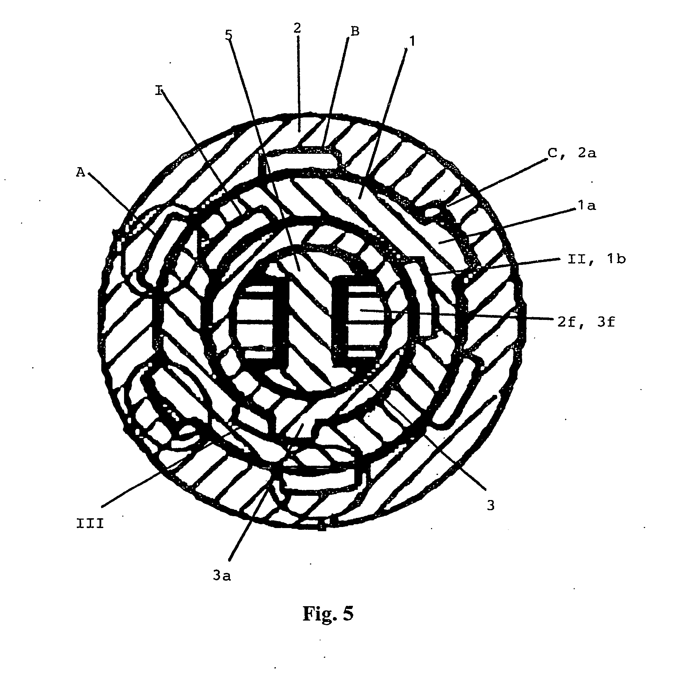

Wie in Figur 5 gezeigt, kann als erstes Gewinde zwischen dem Gehäuse 2 und der Drehhülse 1 einer von mehreren in dem Gehäuse 2 vorgesehenen Gewindegänge A, B oder C gewählt werden. Die Gewinde A, B und C sind im Gehäuse 2 zum Beispiel um cirka 120°, aber beispielsweise nicht genau um 120° verteilt angeordnet.

Die verschiedenen Gewinde I, II und III dienen beispielsweise dazu, die Orientierung der Schnapper 3f gegenüber der Zahnstange 5 zu korrigieren, so dass die Schnapper 3f stets in Führungsrillen passen, welche entlang der Zahnstange vorgesehen und in welchen die Zahnreihen der Zahnstange 5 angeordnet sind. Wurde eines der Gewinde I, II oder III für den Eingriff der Aussengewinde 3a der Vorschubhülse 3 ausgewählt, bilden die Vorschubhülse 3 und die Drehhülse 1 eine Einheit, deren Länge durch die Drehposition der Hülsen 1 und 3 zueinander variiert werden kann. Zum Einsetzen dieser Einheit in das Gehäuse 2 wird das Aussengewinde 1a der Drehhülse 1 in eines der Gewinde A, B oder C eingesetzt. Ist kein Längenausgleich bei der Einheit aus Vorschubhülse 3 und Drehhülse 1 notwendig damit die Schnapper 3f vollständig in die Zahnreihen der Zahnstange 5 eingreifen können, d. h. ist kein Ausgleich aufgrund von Fertigungstoleranzen notwendig, wird das Aussengewinde 1a in dem oben beschriebenen Beispiel einer Versetzung der Gewinde von 118Grad/122Grad in Gewinde C eingesetzt. Ist es jedoch erforderlich, die Länge der Einheit aus Vorschubhülse 3 und Drehhülse 1 zu korrigieren, damit die Schnapper 3f in die Zahnreihen der Zahnstange 5 vollständig eingreifen können, wird das Aussengewinde 1 a in den Gewindegang B oder C des Gehäuses 2 eingesetzt. Hierfür wird die Drehhülse 1 gegenüber dem Gehäuse 2 solange verdreht, bis der Gewindeanfang des Aussengewindes 1a gegenüber dem Gewindeeingang des Gewindes B oder C zu liegen kommt und somit das Aussengewinde 1a in das Gewinde B oder C eingreifen kann. Bei der Drehung der Drehhülse 1 dreht die Vorschubhülse 3 relativ zum Gehäuse 2 nicht mit, so dass sich bei Drehung der Drehhülse 1 die Länge der Einheit aus Vorschubhülse 3 und Drehhülse 1 und somit die Position der Schnapper 3f gegenüber der gehäusefesten Zahnstange 5 verändert, indem die Vorschubhülse 3 axial vor oder zurück verschoben wird.

Als zweites Gewinde zwischen der Drehhülse 1 und der Vorschubhülse 3 kann ebenfalls einer von mehreren in der Drehhülse 1 vorgesehenen Gewindegänge I, II oder III gewählt werden, wobei die Gewinde I, II und III in der Drehhülse zum Beispiel um genau 120° verteilt angeordnet sein können.

Beispielsweise dient das Gewinde A einer negativen Längenkorrektur, das Gewinde B einer positiven Längenkorrektur und das Gewinde C einer Nullkorrektur, wobei Nullkorrektur bedeutet, dass keine Toleranzkorrektur notwendig ist. Hierfür ist zum Beispiel das Gewinde A um 118 Grad und das Gewinde B um 122 Grad auf dem Umfang des Gehäuses 2 gegenüber dem Gewinde C versetzt angeordnet. Natürlich ist auch eine andere Versetzungskombination, wie etwa 119 Grad, bzw. 121 Grad möglich.

Durch eine passende Auswahl einer Gewindekombination kann bei der Montage der Injektionsvorrichtung der Abstand der Schnapper 2f und 3f voneinander im Bezug auf den Zahnabstand Z der Zähne der Zahnstange 5 so angepasst werden, dass außer bei Aufzieh- und Einschiebbewegungen alle Zähne der Schnapper 2f und 3f vollständig in die Verzahnung der Gewindestange 5 eingreifen können, wodurch Fertigungstoleranzen ausgeglichen werden können.

## Patentansprüche

1. Einstellvorrichtung zum Einstellen einer Injektionsvorrichtung zur Verabreichung einer Substanz mit einem ersten Element (1, 2) und mindestens einem weiteren Element (1,3), welches relativ zum ersten Element (1 , 2) bewegbar gelagert ist, wobei das erste Element (1, 2) mindestens zwei zueinander versetzte Gewindegänge (1b, 2b) aufweist, so dass mindestens ein Gewindeelement (1a, 3a) des mindestens einen weiteren Elements (1, 3) nur in einem der mindestens zwei zueinander versetzten Gewindegänge (1 b, 2b) des ersten Elements (1, 2) geführt wird, **dadurch gekennzeichnet, dass** die mindestens zwei zueinander versetzten Gewindegänge (1b, 2b) so angeordnet sind, dass das mindestens eine Gewindeelement (1a, 3a) des mindestens einen weiteren Elements (1, 3) in mindestens zwei verschiedenen Führungspositionen in das erste Element (1, 2) einsetzbar ist.

2. Einstellvorrichtung nach Anspruch 1, **dadurch gekennzeichnet, dass** das erste oder weitere Element eine Drehhülse (1), ein Gehäuse (2) und/oder ein Vorschubglied(3) ist.

3. Einstellvorrichtung nach einem der vorhergehenden Ansprüche, **dadurch gekennzeichnet, dass** die mindestens zwei zueinander versetzten Gewindegänge (1b, 2b) des ersten Elements (1, 2) radial und/oder axial zueinander versetzt sind.

4. Einstellvorrichtung nach einem der vorhergehenden Ansprüche, **dadurch gekennzeichnet, dass** die mindestens zwei zueinander versetzten Gewindegänge (1 b, 2b) des ersten Elements (1, 2) als Innengewinde und/oder Aussengewinde an dem ersten Element (1, 2) vorgesehen sind.

5. Einstellvorrichtung nach einem der vorhergehenden Ansprüche, **dadurch gekennzeichnet, dass** die mindestens zwei zueinander versetzten Gewindegänge (1b, 2b) des ersten Elements (1, 2) als um laufende Nuten oder Nut-Abschnitte und das mindestens eine Gewindeelement (1a, 3a) des mindestens einen weiteren Elementes (1, 3) als umlaufender Steg oder Steg-Abschnitt ausgebildet ist.

6. Einstellvorrichtung nach einem der vorhergehenden Abschnitte, **dadurch gekennzeichnet, dass** das erste Element (1, 2) n Gewinde zur Führung von m Gewindeelemente des mindestens einen weiteren Elements (1, 3) aufweist, wobei n > m > 1 und bevorzugt mg k = n gilt, wobei k eine natürliche Zahl ist.

7. Dosiervorrichtung mit mindestens einer Einstellvorrichtung (1, 2, 3) nach einem der vorhergehenden Ansprüche, **dadurch gekennzeichnet, dass** ein Steg (1a, 3a) eines Gewindes des mindestens einen weiteren Elementes (1, 3) mit einer der Nuten (1b, 2a) des ersten Elements (1, 2) so im Eingriff ist, dass ein vorgegebenes Lageverhältnis zwischen dem ersten Element (1, 2) und dem weiteren Element (1, 3) realisiert werden kann.

8. Dosiervorrichtung nach dem vorhergehenden Anspruch, **dadurch gekennzeichnet, dass** das Lageverhältnis des ersten Elements (1, 2) relativ zum weiteren Element (1, 3) durch die Position mindestens eines Eingriffselementes oder Schnappers (2f, 3f) an dem ersten Element (1, 2) und/oder dem weiteren Element (1, 3), insbesondere einen Abstand zwischen dem Eingriffselementen oder Schnappern (2f, 3f), vorgegeben ist.

9. Verfahren zur Herstellung einer Einstellvorrichtung für eine Injektionsvorrichtung, wobei ein Gewinde (1a, 3a) mindestens eines Dosierelements (1, 3) so in ein zugeordnetes Gewinde (1b, 2a) aus einer Mehrzahl von mindestens zwei Gewinden eines weiteren Dosierelements (1, 2) eingesetzt wird, dass das Gewinde (1a, 3a) des mindestens einen Dosierelements (1, 3) in mindestens zwei verschiedenen Führungspositionen in das weitere Dosierelement (1, 2) eingesetzt wird, dass ein vorgegebenes Lageverhältnis zwischen dem Dosierelement (1, 3) und dem weiteren Dosierelement (1, 2) hergestellt wird.

10. Dosiervorrichtung für eine Injektionsvorrichtung mit einem Einstellelement (4), einer bevorzugt etwa zylinderförmigen Drehhülse (1), welche mit dem Einstellelement (4) verbunden ist und ein Außengewinde (1a) und ein Innengewinde (1b) aufweist, welches die gleiche oder eine andere Steigung als das Aussengewinde (1a) hat, wobei ein Gewinde (1a,1b) der Drehhülse (1) mit einem Gewinde (2a) eines Teils der Injektionsvorrichtung, insbesondere einem Gehäuse (2), im Eingriff steht und das andere Gewinde (1a, 1b) mit einem Gewinde (3a) eines Vorschubglieds (3) im Eingriff steht, so dass eine Einstellbewegung des Einstellelementes (4) von der Drehhülse (1) durch die beiden Gewindeeingriffe (1a, 2a; 1b, 3a) in eine Dosierbewegung des Vorschubgliedes (3) übertragen oder übersetzt oder untersetzt werden kann, **dadurch gekennzeichnet, dass** das Aussengewinde (1a) und/oder das Innengewinde (1b) aus mindestens zwei verschiedenen um die Drehhülse (1) umlaufenden Gewindegängen und/oder das Gewinde (2a) des Teils (2) der Injektionsvorrichtung aus mindestens zwei verschiedenen versetzten um das Gehäuse (2) umlaufenden Gewindegängen besteht und so angeordnet sind, dass das eine Gewinde (1a, 3a) der Drehhülse (1) und/oder Vorschubgliedes (3) in mindestens zwei verschiedenen Führungspositionen in die Drehhülse und/oder in das Gehäuse (1, 2) einsetzbar ist.

11. Dosiervorrichtung nach Anspruch 10, **dadurch gekennzeichnet, dass** die Steigung desjenigen Gewindes (1b) der Drehhülse (1), welches mit dem Vorschubglied (3) im Eingriff steht, kleiner ist als die Steigung des anderen Gewindes (1a) der Drehhülse (1).

12. Dosiervorrichtung nach Anspruch 10, **dadurch gekennzeichnet, dass** die Steigung desjenigen Gewindes (1b) der Drehhülse (1), welches mit dem Vorschubglied (3) im Eingriff steht, grösser ist als die Steigung des anderen Gewindes (1a) der Drehhülse (1).

13. Dosiervorrichtung nach einem der drei vorhergehenden Ansprüche, **dadurch gekennzeichnet, dass** der Unterschied der Gewindesteigung im Bereich von 5 bis 30 oder 10 bis 15 Grad liegt.

14. Dosiervorrichtung nach einem der vier vorhergehenden Ansprüche, **dadurch gekennzeichnet, dass** das Vorschubglied (3) relativ zum Gehäuse (2) verdrehgesichert gelagert ist.

15. Dosiervorrichtung nach einem der fünf vorhergehenden Ansprüche, **dadurch gekennzeichnet, dass** an dem Gehäuse (2) mindestens zwei Innengewinde (2a) vorgesehen sind, wobei das Aussengewinde (1a) der Drehhülse (1) nur in mindestens eins aber nicht in alle Innengewinde (2a) eingreift und das Vorschubglied (3) mindestens ein Aussengewinde (3a) aufweist, das in mindestens eines von der mehreren Innengewinden (1b) der Drehhülse (1) eingreift.

16. Dosiervorrichtung nach einem der sechs vorhergehenden Ansprüche, **dadurch gekennzeichnet, dass** die Drehhülse (1) fest oder drehbar mit dem Einstellelement (4) verbunden ist.

17. Dosiervorrichtung nach einem der sieben vorhergehenden Ansprüche, **dadurch gekennzeichnet, dass** die Gewindesteigungen der Drehhülse (1), des Gehäuses (2) und des Vorschubgliedes (3) so gewählt werden, dass die Gewinde nicht selbsthemmend sind.

18. Dosiervorrichtung nach einem der acht vorhergehenden Ansprüche, **dadurch gekennzeichnet, dass** ein Federelement, insbesondere eine Torsionsfeder vorgesehen ist, welche in oder gegen die Drehrichtung der Drehhülse (1) wirkt.

19. Dosiervorrichtung nach einem der neun vorhergehenden Ansprüche, **dadurch gekennzeichnet, dass** das Aussengewinde (1a) der Drehhülse (1) koaxial und in radialer Richtung zumindest zum Teil überlappend relativ zu dem Innengewinde (1b) der Drehhülse (1) angeordnet ist.

20. Dosiervorrichtung nach einem der zehn vorhergehenden Ansprüche, **dadurch gekennzeichnet, dass** an dem Vorschubglied (3) mindestens eine Rastnase oder Rastnocke (3f) bevorzugt an einem elastischen Element oder Arm (3e) angebracht ist.

21. Dosiervorrichtung nach einem der elf vorhergehenden Ansprüche, **dadurch gekennzeichnet, dass** eine Zahnstange (5) vorgesehen ist, welche axial verschiebbar innerhalb der Dosiervorrichtung und bevorzugt innerhalb des Vorschubgliedes (3) gelagert ist und mindestens eine oder zwei Zahnreihen an der Aussenseite aufweist, in welcher mindestens eine oder zwei Zahnreihen an der Aussenseite aufweist, in welche mindestens eine elastische gelagerte Rastnase (3f) des Vorschubgliedes (3) und/oder mindestens eine elastisch gelagerte Rastnase (2f), welche mit dem Gehäuse (2) verbunden ist, eingreifen kann.

22. Dosiervorrichtung nach einem der zwölf vorhergehenden Ansprüche, **dadurch gekennzeichnet, dass**, mindestens ein radialen und/oder axialen Anschlag (3c, 3d) am Vorschubglied (3) und/oder an der Drehhülse (1) und/oder am Gehäuse (2) vorsehen ist, um eine Radial- und/oder Axialbewegung des Vorschubglieds (3) und/oder der Drehhülse (1) relativ zum Gehäuse (2) zu begrenzen, wobei durch einen Anschlag ein Lageverhältnis zwischen der Drehhülse (1), dem Gehäuse (2) und/oder dem Vorschubglied (3) definiert werden kann.

23. Dosiervorrichtung nach einem der 13 vorhergehenden Ansprüche, **dadurch gekennzeichnet, dass**, auf einer Aussenseite des Einstellelementes (4) und/oder einer Aussenseite der Drehhülse (1) Markierungen zur Anzeige einer eingestellten Dosis aufgebracht sind.

24. Injektionsvorrichtung, in welcher eine abzugebende Substanz enthalten ist oder in einer Ampulle (7) eingelegt werden kann, mit einer Dosiervorrichtung nach einem der 14 vorhergehenden Ansprüche.

## Claims

1. A setting means for setting an injection means for administering a substance, comprising a first element (1, 2) and at least one further element (1, 3) which is displaceably mounted relative to the first element (1, 2), wherein the first element (1, 2) has at least two mutually offset threads (1b, 2b), such that at least one thread element (1a, 3a) of the at least one further element (1, 3) is guided in only one of the at least two mutually offset threads (1b, 2b) of the first element (1, 2), **characterized in that** the at least two mutually offset threads (1b, 2b) are arranged such that the at least one thread element (1a, 3a) of the at least one further element (1, 3) can be inserted in the first element (1, 2) in at least two different guide positions.

2. A setting means according to claim 1, **characterized in that** the first or the further element is a rotary sleeve (1), a housing (2) and/or a forward feed member (3).

3. A setting means according to one of the preceding claims, **characterized in that** the at least two mutually offset threads (1b, 2b) of the first element (1, 2) are mutually offset radially and/or axially.

4. A setting means according to one of the preceding claims, **characterized in that** the at least two mutually offset threads (1b, 2b) of the first element (1, 2) are provided as an internal thread and/or an external thread on the first element (1, 2).

5. A setting means according to one of the preceding claims, **characterized in that** the at least two mutually offset threads (1b, 2b) of the first element (1, 2) are formed as circumferential grooves or groove portions and the at least one thread element (1a, 3a) of the at least one further element (1, 3) is formed as a circumferential web or web section.

6. A setting means according to one of the preceding claims, **characterized in that** the first element (1, 2) has n threads to guide m thread elements of the at least one further element (1, 3), wherein n > m > 1 and preferably m x k = n, wherein k is a natural number.

7. A dosing means having at least one setting means (1, 2, 3) according to one of the preceding claims, **characterized in that** a web (1a, 3a) of a thread of the at least one further element (1, 3) is engaged with one of the grooves (1b, 2a) of the first element (1, 2) such that a predetermined positional relationship can be obtained between the first element (1, 2) and the further element (1, 3).

8. A dosing means according to the preceding claim, **characterized in that** the positional relationship of the first element (1, 2) relative to the further element (1, 3) is determined by the position of at least one engaging element or catch (2f, 3f) on the first element (1, 2) and/or the further element (1, 3), in particular by a distance between the engaging elements or catches (2f, 3f).

9. A method for manufacturing a setting means for an injection means, wherein a thread (1a, 3a) of at least one dosing element (1, 3) is inserted in one associated thread (1b 2a) from a plurality of at least two threads of a further dosing element (1, 2) such that the thread (1a, 3a) of the at least one dosing element (1, 3) is inserted in the further dosing element (1, 2) in at least two different guide positions such that a predetermined positional relationship is obtained between the dosing element (1, 3) and the further dosing element (1, 2).

10. A dosing means for an injection means comprising a setting element (4), a preferably approximately cylindrical rotary sleeve (1), which is connected with the setting element (4) and exhibits an external thread (1a) and an internal thread (1b), which has the same pitch as or a different pitch from the external thread (1a), wherein one thread (1a, 1b) of the rotary sleeve (1) engages with a thread (2a) of a part of the injection means, in particular a housing (2), and the other thread (1a, 1b) engages with a thread (3a) of a forward feed member (3), such that a setting movement of the setting element (4) can be transmitted or translated or geared down from the rotary sleeve (1) via the two meshed threads (1a, 2a; 1b, 3a) to produce a dosing movement of the forward feed member (3), **characterized in that** the external thread (1a) and/or the internal thread (1b) consists of at least two different threads extending circumferentially about the rotary sleeve (1) and/or the thread (2a) of the part (2) of the injection means consists of at least two different threads extending circumferentially about the housing (2) and in an offset manner and are arranged such that the one thread (1a, 3a) of the rotary sleeve (1) and/or forward feed member (3) can be inserted into at least two different guide positions in the rotary sleeve and/or in the housing (1, 2).

11. A dosing means according to claim 10, **characterized in that** the pitch of that thread (1b) of the rotary sleeve (1) which engages with the forward feed member (3) is smaller than the pitch of the other thread (1a) of the rotary sleeve (1).

12. A dosing means according to claim 10, **characterized in that** the pitch of that thread (1b) of the rotary sleeve (1) which engages with the forward feed member (3) is larger than the pitch of the other thread (1a) of the rotary sleeve (1).

13. A dosing means according to one of the three preceding claims, **characterized in that** the difference in the thread pitch is in the region of 5 to 30 or 10 to 15 degrees.

14. A dosing means according to one of the four preceding claims, **characterized in that** the forward feed member (3) is mounted so that it is prevented from rotating relative to the housing (2).

15. A dosing means according to one of the five preceding claims, **characterized in that** at least two internal threads (2a) are provided on the housing (2), wherein the external thread (1a) of the rotary sleeve (1) engages in only at least one but not in all of the internal threads (2a) and the forward feed member (3) has at least one external thread (3a), which engages in at least one of the plurality of internal threads (1b) of the rotary sleeve (1).

16. A dosing means according to one of the six preceding claims, **characterized in that** the rotary sleeve (1) is fixedly or rotatably connected to the setting element (4).

17. A dosing means according to one of the seven preceding claims, **characterized in that** the thread pitches of the rotary sleeve (1), the housing (2) and the forward feed member (3) are selected such that the threads are not self-locking.

18. A dosing means according to one of the eight preceding claims, **characterized in that** a spring element, in particular a torsion spring, is provided, which acts in or against the direction of rotation of the rotary sleeve (1).

19. A dosing means according to one of the nine preceding claims, **characterized in that** the external thread (1a) of the rotary sleeve (1) is arranged coaxially with the rotary sleeve (1) and in the radial direction at least partially overlaps the internal thread (1b) of the rotary sleeve (1).

20. A dosing means according to one of the ten preceding claims, **characterized in that** at least one locking lug or locking cam (3f) is arranged on the forward feed member (3), preferably on an elastic element or arm (3e).

21. A dosing means according to one of the eleven preceding claims, **characterized in that** a toothed rack (5) is provided which is axially displaceable inside the dosing means and is preferably mounted inside the forward feed member (3) and has at least one or two rows of teeth on the external face, wherein at least one or two rows of teeth are on the external face, into which at least one elastically mounted locking lug (3f) of the forward feed member (3) and/or at least one elastically mounted locking lug (2f), which is connected with the housing (2), can engage.

22. A dosing means according to one of the twelve preceding claims, **characterized in that** at least one radial and/or axial stop (3c, 3d) is provided on the forward feed member (3) and/or on the rotary sleeve (1) and/or on the housing (2), to restrict radial and/or axial movement of the forward feed member (3) and/or the rotary sleeve (1) relative to the housing (2), wherein the stop can define a positional relationship between the rotary sleeve (1), the housing (2) and/or the forward feed member (3).

23. A dosing means according to one of the thirteen preceding claims, **characterized in that** markings are provided on the external face of the setting element (4) and/or on the external face of the rotary sleeve (1) to indicate the dose which has been set.

24. An injection means which contains a substance to be dispensed or into which an ampoule (7) can be inserted, having a dosing means in accordance with one of the fourteen preceding claims.

## Revendications

1. Dispositif de réglage visant à régler un dispositif d'injection servant à administrer une substance avec un premier élément (1, 2) et au moins un autre élément (1, 3), lequel est monté de façon mobile par rapport au premier élément (1, 2), le premier élément (1, 2) comprenant au moins deux pas de filetage (1b, 2b) décalés l'un par rapport à l'autre, de sorte qu'au moins un élément de filetage (1a, 3a) du au moins un autre élément (1, 3) est guidé uniquement dans un des au moins deux pas de filetage (1b, 2b) décalés l'un par rapport à l'autre du premier élément (1, 2), **caractérisé en ce que** les au moins deux pas de filetage (1b, 2b) décalés l'un par rapport à l'autre sont disposés de telle sorte que le au moins un élément de filetage (1a, 3a) du au moins un autre élément (1, 3) est insérable dans au moins deux positions de guidage différentes dans le premier élément (1, 2).

2. Dispositif de réglage selon la revendication 1, **caractérisé en ce que** le premier ou l'autre élément est une douille rotative (1), un logement (2) et/ou un élément d'avance (3).

3. Dispositif de réglage selon l'une des revendications précédentes, **caractérisé en ce que** les au moins deux pas de filetage (1b, 2b) décalés l'un par rapport à l'autre du premier élément (1, 2) sont décalés l'un par rapport à l'autre de façon radiale et/ou axiale.

4. Dispositif de réglage selon l'une des revendications précédentes, **caractérisé en ce que** les au moins deux pas de filetage (1b, 2b) décalés l'un par rapport à l'autre du premier élément (1, 2) sont prévus comme le filetage intérieur et/ou le filetage extérieur sur le premier élément (1, 2).

5. Dispositif de réglage selon l'une des revendications précédentes, **caractérisé en ce que** les au moins deux pas de filetage (1b, 2b) décalés l'un par rapport à l'autre du premier élément (1, 2) sont formés comme des rainures ou des sections de rainure rotatives et le au moins un élément de filetage (1a, 3a) du au moins un autre élément (1, 3) est formé comme une nervure ou une section de nervure tournante.

6. Dispositif de réglage selon l'une des revendications précédentes, **caractérisé en ce que** le premier élément (1, 2) comprend n filetages servant à guider m éléments de filetage du au moins un autre élément (1, 3), sachant que n > m > 1 et que, de préférence, m.k = n, k étant un nombre naturel.

7. Dispositif de dosage doté d'au moins un dispositif de réglage (1, 2, 3) selon l'une des revendications précédentes, **caractérisé en ce qu'**une nervure (1a, 3a) d'un filetage du au moins un autre élément (1, 3) est en prise avec une des rainures (1b, 2a) du premier élément (1, 2) de sorte qu'il est possible d'obtenir un rapport de positionnement prédéfini entre le premier élément (1, 2) et l'autre élément (1, 3).

8. Dispositif selon la revendication précédente, **caractérisé en ce que** le rapport de positionnement du premier élément (1, 2) par rapport à l'autre élément (1, 3) est prédéfini par la position d'au moins un élément de maintien ou d'un dispositif d'accouplement à clipsage (2f, 3f) sur le premier élément (1, 2) et/ou l'autre élément (1, 3), en particulier une distance entre l'élément de maintien ou le dispositif d'accouplement à déclic (2f, 3f).

9. Procédé de fabrication d'un dispositif de réglage pour un dispositif d'injection, un filetage (1a, 3a) d'au moins un élément (1, 3) étant emboîté dans un filetage affecté (1b, 2a) à partir d'une pluralité d'au moins deux filetages d'un autre élément (1, 2) de sorte que le filetage (1a, 3a) du au moins un élément de dosage (1, 3) est emboîté dans au moins deux positions de guidage différentes dans l'autre élément de dosage (1, 2), de sorte qu'un rapport de positionnement prédéfini entre l'élément de dosage (1, 3) et l'autre élément de dosage (1, 2) est réalisé.

10. Dispositif de dosage pour un dispositif d'injection avec un élément de réglage (4), une douille rotative (1) de préférence par exemple cylindrique, lequel est relié à l'élément de réglage (4) et comprend un filetage extérieur (1a) et un filetage intérieur (1b), lequel possède un pas identique ou différent à celui du filetage extérieur (1a), un filetage (1a, 1b) de la douille rotative (1) étant en prise avec un filetage (2a) d'une partie du dispositif d'injection, en particulier d'un logement (2), et l'autre filetage (1a, 1b) étant en prise avec un filetage (3a) d'un élément d'avance (3), de sorte qu'un mouvement de réglage de l'élément de réglage (4) peut être transféré ou multiplié ou démultiplié à partir de la douille rotative (1) à travers les deux engagements de filetage (1a, 2a ; 1b, 3a) dans un mouvement de dosage de l'élément d'avance (3), **caractérisé en ce que** le filetage extérieur (1a) et/ou le filetage intérieur (1b) se compose d'au moins deux pas de filetage différents situés autour de la douille rotative (1) et/ou le filetage (2a) de la pièce (2) du dispositif d'injection se compose d'au moins deux pas de filetage différents décalés situés autour du logement (2) et qui sont disposés de telle sorte que le un filetage (1a, 3a) de la douille rotative (1) et/ou de l'élément d'avance (3) est insérable dans au moins deux positions de guidage différentes dans la douille rotative et/ou dans le logement (1, 2).

11. Dispositif de dosage selon la revendication 10, **caractérisé en ce que** le pas du filetage (1b) de la douille rotative (1), qui est en prise avec l'élément d'avance (3), est plus petit que le pas de l'autre filetage (1a) de la douille rotative (1).

12. Dispositif de dosage selon la revendication 10, **caractérisé en ce que** le pas du filetage (1b) de la douille rotative (1), qui est en prise avec l'élément d'avance (3), est plus grand que le pas de l'autre filetage (1a) de la douille rotative (1).

13. Dispositif de dosage selon l'une des trois revendications précédentes, **caractérisé en ce que** la différence de pas de filetage se trouve dans un secteur compris entre 5 et 30 degrés ou entre 10 et 15 degrés.

14. Dispositif de dosage selon l'une des quatre revendications précédentes, **caractérisé en ce que** l'élément d'avance (3) est logé sans possibilité de rotation par rapport au logement (2).

15. Dispositif de dosage selon l'une des cinq revendications précédentes, **caractérisé en ce qu'**au moins deux filetages intérieurs (2a) sont prévus sur le logement (2), le filetage extérieur (1a) de la douille rotative (1) venant en prise uniquement dans au moins un filetage intérieur mais pas dans tous (2a) et **en ce que** l'élément d'avance (3) comprend au moins un filetage extérieur (3a) qui vient en prise dans au moins un de la pluralité filetages intérieurs (1b) de la douille rotative (1).

16. Dispositif de dosage selon l'une des six revendications précédentes, **caractérisé en ce que** la douille rotative (1) est reliée de façon fixe ou pivotante à l'élément de réglage (4).

17. Dispositif de dosage selon l'une des sept revendications précédentes, **caractérisé en ce que** les pas de filetage de la douille rotative (1), du logement (2) et de l'élément d'avance (3) sont choisis de telle sorte que les filetages ne soient pas autobloquants.

18. Dispositif de dosage selon l'une des huit revendications précédentes, **caractérisé en ce qu'**un élément de ressort, en particulier un ressort de torsion, est prévu pour agir dans le sens de rotation de la douille rotative (1) ou dans le sens opposé.

19. Dispositif de dosage selon l'une des neuf revendications précédentes, **caractérisé en ce que** le filetage extérieur (1a) de la douille rotative (1) est disposé de façon coaxiale et dans une direction radiale, au moins en partie en recouvrement par rapport au filetage intérieur (1b) de la douille rotative (1).

20. Dispositif de dosage selon l'une des dix revendications précédentes, **caractérisé en ce qu'**au moins un ergot de crantage ou un ergot d'enclenchement (3f) est monté sur l'élément d'avance (3), de préférence sur un élément ou un bras flexible (3e).

21. Dispositif de dosage selon l'une des onze revendications précédentes, **caractérisé en ce qu'**une crémaillère (5) est prévue, qui est logée de façon à pouvoir être déplacée de manière axiale dans le dispositif de dosage et de préférence dans l'élément d'avance (3) et qui comprend au moins une ou deux rangées de dents sur le côté extérieur, dans lequel au moins une ou deux rangées de dents est comprise(s) sur le côté extérieur, dans lequel au moins un ergot de crantage (3f) de l'élément d'avance (3) et/ou au moins un ergot de crantage (2f) logé de façon flexible, qui est relié au logement (2), peut venir en prise.

22. Dispositif de dosage selon l'une des douze revendications précédentes, **caractérisé en ce qu'**au moins une butée radiale et/ou axiale (3c, 3d) est prévue sur l'élément d'avance (3) et/ou sur la douille rotative (1) et/ou sur le logement (2), afin de limiter un mouvement radial et/ou axial de l'élément d'avance (3) et/ou de la douille rotative (1) par rapport au logement (2), un rapport de positionnement entre la douille rotative (1), le logement (2) et/ou l'élément d'avance (3) pouvant être défini par une butée.

23. Dispositif de dosage selon l'une des treize revendications précédentes, **caractérisé en ce que** des repères servant à indiquer une dose programmée sont imprimés sur un côté extérieur de l'élément de réglage (4) et/ou un côté extérieur de la douille rotative (1).

24. Dispositif d'injection, dans lequel une substance à administrer est contenue ou peut être inséré dans une ampoule (7), avec un dispositif de dosage selon l'une des 14 revendications précédentes.
